(19) **Europäisches Patentamt**
**European Patent Office**
**Office européen des brevets**

(11) **EP 4 510 938 B1**

(12) **EUROPEAN PATENT SPECIFICATION**

(45) Date of publication and mention
of the grant of the patent:
**24.12.2025 Bulletin 2025/52**

(21) Application number: **23718303.3**

(22) Date of filing: **19.04.2023**

(51) International Patent Classification (IPC):
*A61B 8/08* $^{(2006.01)}$       *A61B 8/00* $^{(2006.01)}$
*G01S 7/539* $^{(2006.01)}$

(52) Cooperative Patent Classification (CPC):
**A61B 8/08; A61B 8/42; A61B 8/4483; A61B 8/485;**
**A61B 8/5207; G01S 7/52042; G01S 15/8909;**
**G01S 15/8925**

(86) International application number:
**PCT/EP2023/060089**

(87) International publication number:
**WO 2023/203056 (26.10.2023 Gazette 2023/43)**

(54) **METHOD AND SYSTEM FOR DETERMINING THE VELOCITY OF A NATURAL SHEAR WAVE PROPAGATING IN A MEDIUM**

VERFAHREN UND SYSTEM ZUR BESTIMMUNG DER GESCHWINDIGKEIT EINER SICH IN EINEM MEDIUM AUSBREITENDEN NATÜRLICHEN SCHERWELLE

PROCÉDÉ ET SYSTÈME DE DÉTERMINATION DE LA VITESSE DE PROPAGATION D'UNE ONDE DE CISAILLEMENT NATURELLE DANS UN MILIEU

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **19.04.2022 EP 22305570**

(43) Date of publication of application:
**26.02.2025 Bulletin 2025/09**

(73) Proprietors:
• **Institut National de la Santé et de la Recherche Médicale**
**75013 Paris (FR)**
• **Centre National de la Recherche Scientifique**
**75016 Paris (FR)**
• **Ecole Supérieure de Physique et de Chimie Industrielles de la Ville de Paris**
**75005 Paris (FR)**

(72) Inventors:
• **PAPADACCI, Clément**
**75012 PARIS (FR)**
• **PERNOT, Mathieu**
**75012 PARIS (FR)**
• **TANTER, Mickael**
**75012 PARIS (FR)**

(74) Representative: **Plasseraud IP**
**104 Rue de Richelieu**
**CS92104**
**75080 Paris Cedex 02 (FR)**

(56) References cited:
• **CHENGWU HUANG ET AL: "Three-dimensional shear wave elastography on conventional ultrasound scanners with external vibration", PHYSICS IN MEDICINE AND BIOLOGY, INSTITUTE OF PHYSICS PUBLISHING, BRISTOL GB, vol. 65, no. 21, 2 November 2020 (2020-11-02), pages 215009, XP020357980, ISSN: 0031-9155, [retrieved on 20201102], DOI: 10.1088/ 1361-6560/ABA5EA**
• **BERNAL MIGUEL ET AL: "Towards 3D passive shear elasticity imaging using row-columns arrays", 2021 IEEE INTERNATIONAL ULTRASONICS SYMPOSIUM (IUS), IEEE, 11 September 2021 (2021-09-11), pages 1 - 4, XP034018447, DOI: 10.1109/ IUS52206.2021.9593576**

- **GENNISSON JEAN-LUC ET AL: "4-D ultrafast shear-wave imaging", IEEE TRANSACTIONS ON ULTRASONICS, FERROELECTRICS, AND FREQUENCY CONTROL, IEEE, USA, vol. 62, no. 6, 1 June 2015 (2015-06-01), pages 1059 - 1065, XP011583941, ISSN: 0885-3010, [retrieved on 20150608], DOI: 10.1109/TUFFC.2014.006936**

## Description

### Technical Field

The present disclosure relates to ultrasound measurement techniques, and particularly, method and device for determining the velocity of a natural shear wave propagating in a medium.

### Background Art

Nowadays, the interest to use ultrasound waves in the medical field no longer needs to be proven.

Indeed, the study of mechanical waves propagating in a medium may allow to retrieve the properties of this medium. For instance, in the medical field, the study of the mechanical wave (e.g. compression and/or shear wave.) propagating in biological tissues (of an organ for instance) may allow to retrieve the elastic properties of this biological tissue, and then determine the state of the organ for instance. Indeed, the state of an organ as a heart may be related to its elastic properties such the rigidity.

Usually, the elastic properties may be determined based on the velocity of the mechanical wave in the medium. Because the compression waves propagate too fast in the medium, it is generally preferred to use the shear waves for determining the elastic properties of medium. The shear wave propagating may be generated artificially by an ultrasound probe (or ultrasound transducer) which may then detect it or may be generated by a natural source as the activity of organs. The main drawback when using artificial shear waves is the need of high-cost system suitable to generate the shear wave in the medium and detect its propagation with high precision, requirements needed for determining correctly its velocity and therefore the elastic properties of the biological tissues. The main drawback when using the natural shear waves for determining the elastic properties is the difficulty to measure such natural shear wave in all dimensions, requirements for determining with high precision the velocity and therefore the elastic properties. Indeed, contrary to artificial shear waves, by nature, a source of natural shear wave is uncontrolled in terms of location, wavelength and amplitude. The propagation of the shear wave from the source occurs in three dimensions along unknown directions. Accordingly, the quantification of elastic properties by using shear wave propagation requires a complex three-dimensional inverse problem which is generally simplified in two dimensions. Such simplification impacts the precision in the determination of the velocity and therefore the precision of the elastic properties of the biological tissues.

Therefore, there is a need to determine with high precision the velocity of a natural shear wave propagating in a medium.

The publication "Three-dimensional shear wave elastography on conventional ultrasound scanners with external vibration" by Chengwu Huang et al., Physics in Medicine and Biology, Institute of Physics Publishing, Bristol, GB, vol. 65, no. 21, p. 215009, XP020357980, discloses a method for determining a velocity of a shear wave propagating in a medium, said shear wave having a wavelength. In the method, a sinusoidal shear wave is generated in the medium and the shear wave is three-dimensionally tracked with an ultrasound scanner using a sub-Nyquist sampling frequency. The aliasing is suppressed by sophisticated techniques, allowing for a three-dimensional shear wave speed reconstruction. The shear wave speed $c_s$ is finally calculated based on the vibration frequency f0 and the spatial wavelength $\lambda$ ($c_s = f_0\lambda$).

### Summary

To this end, the present invention is directed at a method according to claim 1.

Advantageously, it may be possible to determine the global velocity of a shear wave propagating in a medium in a simple and efficiency way without the need to complex imaging systems and methods. Furthermore advantageously, it may be possible to determine the elastic properties of the medium based on the determined velocity of the shear wave.

By illuminating, it may be understood an observation point comprised in the medium, which is crossed by the at least one ultrasound beam.

The vector may be expressed in polar, cylindrical or spherical coordinates.

The at least one ultrasound beam may be focused or defocused.

In one or several embodiments, the plurality of backscattered signals may be respectively acquired by a plurality of ultrasound transducers, each ultrasound transducer acquiring backscattered signals backscattered respectively from a respective observation point.

Preferably, in one or several embodiments, the plurality of backscattered signals may be respectively acquired by at least three ultrasound transducers of the plurality of ultrasound transducers, each ultrasound transducer acquiring backscattered signals backscattered respectively from a respective observation point.

Advantageously, the more there are observation points and ultrasound transducers for acquiring backscattered signals backscattered respectively from a respective observation point, and the more precision on the component along X and Y axes (or axis) are increased, and therefore, the precision on the velocity of the shear wave.

In one or several embodiments, the respective observation points may be separated from each other by a separation distance less than the wavelength of the shear wave in the medium.

Advantageously, such separation distance allows to consider the shear wave as a plane shear wave.

In one or several embodiments, at least three ultrasound beams may be generated into the medium illuminating respectively the at least three respective observation points in the medium.

In one or several embodiments, the at least three ultrasound beams may be generated respectively by the plurality of ultrasound transducers.

In one or several embodiments, the at least three ultrasound beams may be generated respectively by at least three ultrasound transducers of the plurality of ultrasound transducers.

Each ultrasound beam of the at least three ultrasound beams may be focused or defocused.

In one or several embodiments, when the wave vector direction of the shear wave is known, the at least three observation points may be not aligned in the plane that contains the wave vector of the shear wave, or when the wave vector direction of the shear wave is unknown, the at least three observation points may be at least four observation points which are not aligned and not all comprised in a same plane.

In one or several embodiments, the wavelength of the shear wave may be less than 0.7 meters, preferably less than 0.5 meters.

In one or several embodiments, each arrival time of the plurality of respective arrival times for a respective observation point may be determined using a motion estimator on backscattered signals respectively acquired from this respective observation point.

The motion estimator may be used on the backscattered signals to determine a tissue displacement or/and a tissue velocity.

In one or several embodiments, each arrival time of the plurality of respective arrival times for a respective observation point may be determined based on a respective variation of a tissue velocity determined by the motion estimator at this respective observation point.

Alternatively, the respective variation may be a respective variation of tissue displacement.

In one or several embodiments, each arrival time of the plurality of respective arrival times for a respective observation point may be determined using directly the acquired backscattered signal(s) or the radio signal frequency respectively acquired from this respective observation point.

In one or several embodiments, the variation of the tissue velocity at a respective observation point may be computed from a plurality of images constructed from the backscattered signals respectively acquired from this respective observation point.

In one or several embodiments, calculating the vector velocity components of the shear wave may further comprise a determination of a plurality of at least three delays, each delay ($\Delta t$) may be determined between two respective arrival times of the plurality of arrival times, and each vector velocity component may be function of a respective delay ($\Delta t$) of the plurality of at least three delays.

In one or several embodiments, each vector velocity component may further be function of the positions of each observation point or lengths between each observation point.

In one or several embodiments, each observation point may be defined by a respective depth in the medium.

In one or several embodiments, the depth of at least one observation points (S'; M'; P') in the medium may be swept along a transmission direction Z0.

Advantageously, such disposition allows to increase the precision on the velocity component along Z axis (parallel to the transmission direction and central axis of the ultrasound beams), and therefore the precision on the velocity of the shear wave.

In one or several embodiments, each arrival time of a respective observation point determined based on the respective variation of the tissue velocity at this respective observation point may be determined by detecting a maximum and/or a minimum of the respective variation or by using crossed correlation or artificial intelligence algorithm.

In one or several embodiments, estimating of the velocity of the shear wave based on the calculated vector velocity components may be performed by using inversion algorithm on the calculated vector velocity components.

Of course, other known techniques may be used to calculate the vector velocity components such function minimization, or regularization methods or artificial intelligence algorithms.

In one or several embodiments, the plurality of ultrasound transducers may be arranged in a 2D array.

In one several embodiments, when at least one of the at least three ultrasound beams is focused, the observation point (S', M', P') may be defined along a transmission direction Z0 so that to be located in a focus spot of the focused ultrasound beam illuminating the observation point.

In one or several embodiments, the shear wave may be a natural shear wave generated by a natural source of shear waves comprised in the medium or is an artificial shear wave generated by an artificial source of shear waves outside the medium.

In one or several embodiments, the natural source of shear waves may be a movement of closure valves of a heart or vibration caused by voice or pulse wave propagating in the arterial wall.

In one or several embodiments, the plurality of ultrasound transducers may be located on the surface of the medium and/or into the medium.

In one or several embodiments, the ultrasound transducers of the plurality of ultrasound transducers may be at distance comprised between 0.001 and 0.1 meters from the natural source of shear waves.

In one or several embodiments, the at least three ultrasound transducers may be at least four ultrasound transducers.

The present invention is further directed at a detection system according to claim 13.

In one or several embodiments, the at least one ultrasound beam may be formed by transmitting a plurality of ultrasound waves generated by a respective ultrasound transducer of the plurality of ultrasound transducers in the medium.

In one or several embodiments, the ultrasound waves may be transmitted at a rate comprised between 100 and 30000 per second.

In one or several embodiments, the frequency of the at least one ultrasound beam may be comprised between 0.5 and 100 MHz.

In one or several embodiments, at least three ultrasound beams may be generated into the medium illuminating respectively the at least three respective observation points (S', M', P') in the medium.

In one or several embodiments, the at least three ultrasound beams may be generated by the plurality of ultrasound transducers.

In one or several embodiments, each ultrasound transducer of the plurality of ultrasound transducers may acquire backscattered signals backscattered respectively from a respective observation point.

Alternatively, or preferably, in one or several embodiments, each ultrasound transducer of the plurality of ultrasound transducers may acquire backscattered signals backscattered respectively from only one respective observation point.

In one or several embodiments, when the wave vector direction of the shear wave is known, the at least three observation points may be not aligned in the plane that contains the wave vector of the shear wave, or when the wave vector direction of the shear wave is unknown, the at least three observation points may be at least four observation points which are not aligned and not all comprised in a same plane.

In one or several embodiments, the ultrasound transducers of the plurality of ultrasound transducers generating respectively the at least three ultrasound beams may be not aligned between each other.

In one or several embodiments, the shear wave may be a natural shear wave generated by a natural source of shear waves comprised in the medium or is an artificial shear wave generated by an artificial source of shear waves outside the medium.

In one or several embodiments, the natural source of shear waves may be a movement of closure valves of a heart or vibration caused by voice or pulse wave propagating in the arterial wall.

In one or several embodiments, the at least three ultrasound transducers may be at least four ultrasound transducers.

In or several embodiments, the plurality of ultrasound transducers may be located on the surface of the medium and/or into the medium.

In one or several embodiments, the plurality of the ultrasound transducers arranged in 2D array may be comprised in an ultrasound probe.

In one or several embodiments, each ultrasound transducer may be formed by a matrix of transducer elements adapted to be controlled independently, and the control system may be configured to control the ultrasound transducer elements so that to emulate an ultrasound transducer generating an ultrasound beam in the medium.

**Brief Description of Drawings**

Other features, details and advantages will be shown in the following detailed description and on the figures, on which:

### Fig. 1

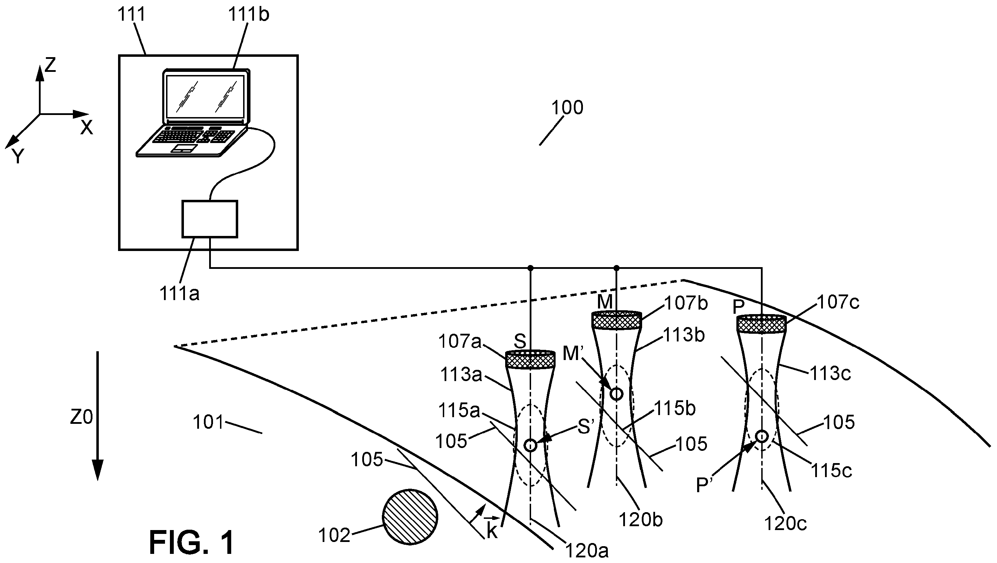

[Fig. 1] illustrates schematically an overall schematic view of a detection system according to the present disclosure.

### Fig. 2a

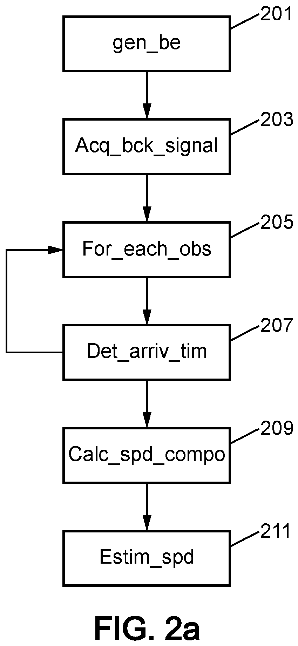

[Fig. 2a] illustrates the flowchart of the method according to the present disclosure.

### Fig. 2b

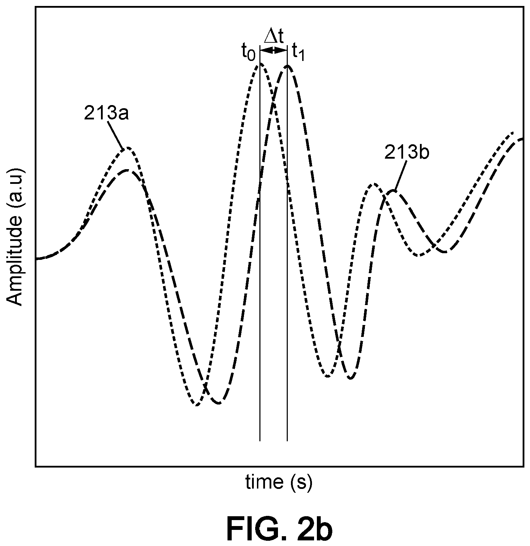

[Fig. 2b] illustrates the principle of detection of two arrival times.

**Fig. 2c&2d**

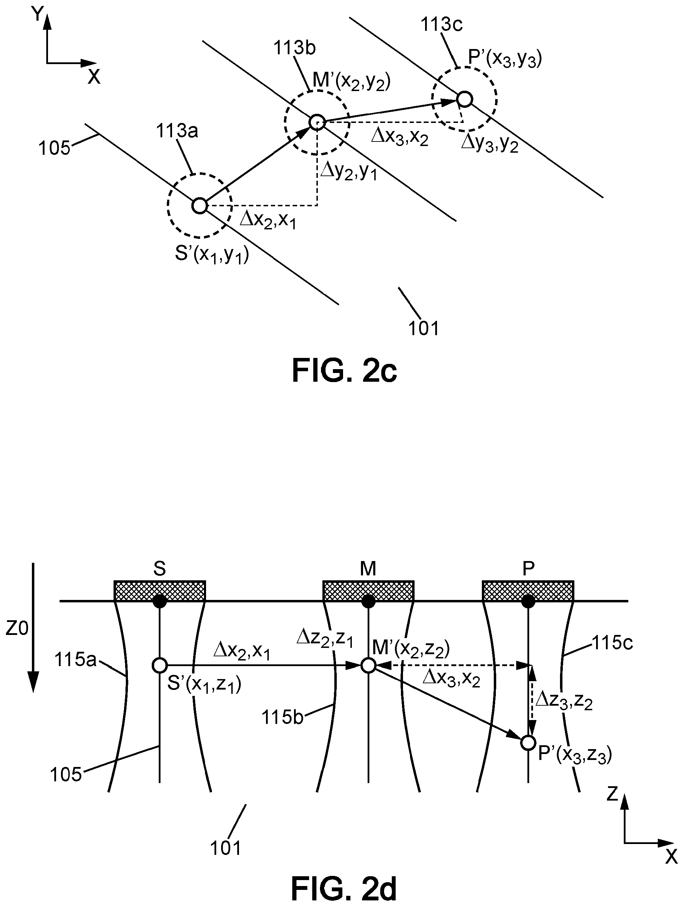

[Fig. 2c and Fig. 2d] illustrate respectively the propagation of a shear wave in a medium according to a XY plane and a XZ plane.

**Fig. 3a**

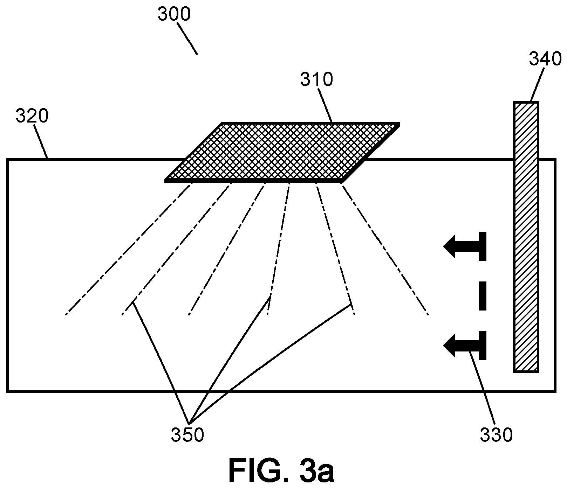

[Fig. 3a] illustrates an experimental setup for measuring the velocity of a shear wave propagated in a medium.

**Fig. 3b**

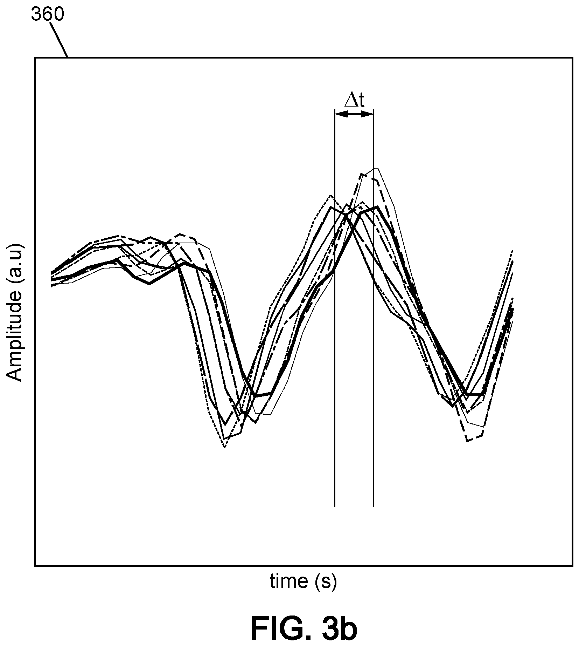

[Fig. 3b] presents the results of the variations of tissue velocity signals determined based on the backscattered signals respectively acquired by the experimental setup from observation points.

**Description of Embodiments**

Figure 1 illustrates schematically an overall schematic view of a detection system according to the present disclosure.

In the various figures, the same references designate identical or similar items.

The detecting system 100 shown on figure 1 may be capable of detecting a shear wave such a natural shear wave or an artificial shear wave propagating in a medium 101, for instance an organ comprised in a chest of a patient, and capable of determining (or evaluate) the global velocity (or speed) of the shear wave(s).

The medium (e.g. organ or a medium comprising an organ) may comprise a shear wave source 102 allowing to generate shear waves 105 propagating in the medium in all directions (x,y,z). The source of shear waves may be a natural source of shear waves (or an artificial source of shear waves) allowing to generate natural shear waves (or artificial shear waves when using artificial source) in a medium. According to an example, the natural source of shear waves may correspond the movement of the heart valves such the closure of the valves, or the contraction of the atria of the heart or the electrotechnical wave propagation.

In one or several embodiments, the source of shear wave may be an artificial source of shear waves outside the medium suitable to generate shear wave in a medium, such an artificial vibration source at a frequency between 1 and 1000 Hz.

The detection system 100 may comprise a plurality (e.g. at least three ultrasound transducers or at least four ultrasound transducers) of ultrasound transducers 107a, 107b, 107c located respectively at positions S, M, P around the source of shear waves, for instance a natural source of shear waves, and a control system 111. Each ultrasound transducer 107a, 107b, 107c may be configured for generating an respective ultrasound beam 113a, 113b, 113c in the medium illuminating a respective observation point S',M',P' (but not necessarily) defined in the medium (defined by the detection system for instance) according to respective coordinates (x, y, z). For instance, at least three (or at least four for instance) ultrasound transducers may generate respectively at least three ultrasound beams. For instance, the ultrasound transducer 107a may generate the ultrasound beam 113a, the ultrasound transducer 107b may generate the ultrasound beam 113b, and the ultrasound transducer 107c may generate the ultrasound beam 113c.

Furthermore, each ultrasound transducer 107a; 107b; 107c may be configured for acquiring (or receiving) a respective backscattered signal generated in response to an interaction between the medium, at respective observation points S',M',P', and the respective ultrasound beam 113a, 113b, 113c generated by the ultrasound transducer 107a, 107b, 107c.

For instance, the ultrasound transducer 107a may generate the ultrasound beam 113a and may acquire the backscattered signal generated by the interaction between the ultrasound beam 113a and the medium 101 at the respective observation point S' in the medium.

In one or several embodiments, only one ultrasound transducer of the plurality of transducers may be used to generate an ultrasound beam so that to illuminate in the same time, the observation points (e.g. at least two or three or more) comprised in the medium while the plurality of ultrasound transducers (e.g. at least two or three ultrasound transducers) may acquire the plurality of backscattered signals backscattered respectively from said observation points illuminated by the ultrasound beam. For instance, the ultrasound transducer 107b of the plurality of ultrasound transducers may generate the ultrasound beam 113b illuminating in the same time the respective observation points S',M',P'. Each ultrasound transducer 107a; 107b; 107c may then acquire the respective backscattered signal generated in response to the interaction between the medium, at respective observation points S',M',P', and the ultrasound beam 113b generated by the ultrasound transducer 107b.

In one or several embodiments, it may be used, for instance, two ultrasound transducers or more of the plurality of ultrasound transducers for illuminating three or more (e.g. four) observation points. For instance, a first ultrasound transducer 107a may be used to illuminate the observation points S' and M', and the second ultrasound transducer 107c may be used to illuminate the observation point P' (or the observation point P' and another observation points when more observation points are defined).

Each ultrasound beam 113a; 113b;113c may be all parallel to a same transmission direction (Z0), but not necessarily (for instance they may be inclined along the transmission direction), and may respectively be formed (or generated) by transmitting, into the medium, a plurality of ultrasound waves generated by the respective ultrasound transducer 107a, 107b, 107c comprised in the plurality of ultrasound transducers. Further, the ultrasound beams 115a, 115b, and 115c may be generated so that they are distinct from each other and do not overlap between them.

Each illuminated observation points S', M', P' may be defined (by the control system for instance) so that its coordinates (x,y) in a XY plane correspond to the coordinates of the ultrasound transducer (x,y) acquiring the backscattered signal backscattered from this illuminated observation point (or the ultrasound transducer having illuminated the observation when each observation is illuminated by a respective ultrasound beam)..

For instance, the observation position S' may have the same coordinates, in the plane XY, as the coordinates of the ultrasound transducer located at point S in the plane XY used to acquire the backscattered signal backscattered from the illuminated observation point S'.

Furthermore, each illuminated observation point S', M', P' may be defined so that its coordinate (z) may be defined by a depth in the medium. For instance, the depth may be defined along the transmission direction Z0 or along the central axis 120a;120b;120c of the ultrasound transducer used to acquire the backscattered signal from the illuminated observation point. For instance, the coordinate z for the observation point S' may be defined along the central axis 120a of the ultrasound transducer 107a.

Furthermore, the respective observation points may be comprised in a same plane or not all in the same plane, and/or not aligned between them. Indeed, depending on the type of source of shear wave and its location on or in the medium, such plane may be a plane which contains the wave vector direction of the shear wave) and may be known in advance. Also, such plane may be the plane which may contains the wave vector of the shear wave when the wave vector direction of the shear wave is known. For instance, the plane containing the wave vector may be an inner wall of the heart. In such case, when the wave vector direction of the shear wave is known, three observations points may be enough (e.g. with three ultrasound transducers) to determine the velocity of the shear wave in the medium. On the contrary, when seeking to determine the velocity of shear wave in a medium which may propagate along unknown plane and direction or/and when the wave vector direction of the shear wave is unknown, it may be preferably (but not necessarily) to use at least four observations points (e.g. with at least four ultrasound transducers) which are not aligned and not all comprised in a same plane.

This misalignment (e.g. in the same plane) between the observation points may be carried out by positioning the plurality of ultrasound transducers used to acquire the plurality of backscattered signals so that they are misaligned between each other in a XY plane (e.g. perpendicular to the transmission direction Z0 of the ultrasound transducers) since as described previously, the observation points may have the same coordinates in a XY plane as the ultrasound transducers.

For instance, the ultrasound transducers 107a, 107b, and 107c may be located on the surface of the medium 101 around or/and at proximity of the natural source of shear waves and so that they are not aligned between each other in a XY plane (e.g. perpendicular to the transmission direction Z0). For instance, the ultrasound transducer 107a may be located at a first position $S(x_1,y_1)$, the ultrasound transducer 107b may be located at a second position $M(x_2,y_2)$, and the ultrasound transducer 107c may be located at a third position $P(x_3,y_3)$ with different coordinates for each of position. Such disposition may allow to have the plurality of respective ultrasound beams, or the respective observation points misaligned between them in a XY plane (e.g. perpendicular to the transmission direction Z0).

Such misalignment may allow to determine with a high precision each component x and y of the velocity of the shear wave needed to determine with a high precision the velocity of the shear wave.

Besides, the respective observation points S',M', P' (or the ultrasound beams 115a, 115b, 115c) may be separated from each other by a separation distance less than the wavelength of the shear wave. Such constraint of the separation distance may allow to make the assumption that the shear wave propagating in the medium may be considered as a wave plane. For instance, the separation distance may less than 0.7 meters, and preferably less than 0.3 meters. Indeed, the shear wave may generally present a wavelength less than 0.7 meter in the medium.

The frequency of the ultrasound waves (or central frequency) or ultrasound beams may be comprised, for instance, between 0.5 and 100 MHz, or for instance between 1 and 10 MHz.

In one or several embodiments, the plurality of ultrasound transducers 107a, 107b, 107c may comprise a number of transducers comprise between 3 and 100 transducers. The ultrasound transducers may be defined by a diameter comprised between 0.05 and 10 centimeters.

Furthermore, the ultrasound beam may be focused or defocused. In this purpose, the ultrasound transducer may present a concavity of its surface so that to allow the focus of the ultrasound beam in the medium and generate a focus spot in the medium. Thus, each focused ultrasound beam 113a, 113b, 113c may comprise a respective focal spot 115a, 115b, 115c which may be under an ellipse form. For instance, the dimensions of the focus spot, at a given depth, may have a diameter along X axis comprised between 0.1 and 10 millimeters and along axis Z comprised between 0.1 and 10 millimeters. When one or all ultrasound beams are focused, the respective observation points S',M',P' may be defined, but not necessarily, so that each respective observation point or at least one among them is located, along the transmission direction, in a respective focal spot 115a, 115b, 115c of the focused ultrasound beam illuminating the observation point. For instance, for the respective observation point S', the coordinate Z may be defined so that to be in the focal spot 115a of the focused ultrasound beam 113a.

The plurality of ultrasound transducers may be located around the source of shear waves or around a plurality of sources of shear waves comprised in the medium. For instance, the plurality of ultrasound transducers 107a; 107b; 107c may be located on the surface of the medium 101 so that to be at proximity of a natural source(s) 102 of shear waves as the closure valves of a heart of a patient. For instance, the ultrasound transducers may be at a distance (i.e. proximity distance) comprised between 0.001 and 0.1 meters from the natural source of shear waves.

According to one or several examples, the plurality of ultrasound transducers may be located (on the surface of the medium or into the medium) along a trajectory (e.g. propagation direction or trajectory) that the shear wave may follow as for instance, along of an artery, or along of the myocardium of the heart.

In one or several embodiments, the plurality of ultrasound transducers may be comprised directly into the medium and located around a natural source(s) of shear waves. For instance, the plurality of transducers may be positioned in an endovascular way so that to be around/at proximity of the organ comprising the natural source of shear waves. For instance, the ultrasound transducers may be at a distance (i.e. proximity distance) comprised between 0.001 and 0.1 meters from the natural source of shear waves.

In one or more embodiments, some transducers of the plurality of transducers may be located on the surface of the medium and some other transducers of the plurality of transducers may be located into the medium (i.e. under the surface of the medium and/or close to a natural source(s) of shear waves).

In one or several embodiments, the plurality of transducers may be comprised in a matrix (e.g. 2D) of transducers integrated in an ultrasound probe. The ultrasound probe may be located on the surface of the medium around/at proximity to the natural source of shear waves and some transducers of the matrix of transducers are used to generate respectively the plurality of ultrasound beams in the medium.

In one or several embodiments, each ultrasound transducer may be integrated in a respective ultrasound probe located on the surface of the medium. For instance, at least three ultrasound probes may be located on the surface of the medium, each ultrasound probe comprising a single or a plurality of transducers which may be independently controlled (by the control system for instance).

In one or several embodiments, each transducer may be formed by a matrix of transducer elements adapted to be controlled independently, and the control system may be configured to control the transducer elements so that to emulate an ultrasound transducer generating an ultrasound beam in the medium.

The control system may be programmed (or configured) such that the ultrasonic waves transmitted by each ultrasound transducer 107a,107b,107c (for formed the ultrasound beams in the medium) may be transmitted at a rate more than 100 ultrasonic waves per second, for instance hundreds to several thousands of ultrasonic waves per second. The control system may for instance include a control unit 111a and a computer 111b. In this example, the control unit 111a may be used for controlling each ultrasound transducer 107a, 107b, 107c, while the computer 111b may be used for controlling the control unit 111a, for generating at least one ultrasound beam, for acquiring backscattered signal(s) by each ultrasound transducer, for detecting a variation based on the acquired backscattered signal(s), for calculating the components of velocity of the shear wave (or the vector velocity components of the shear wave), and for estimating the velocity of the shear wave. In a variant, a single electronic device could fulfill all the functionalities of control unit 111a and computer 111b.

According to an example, the control system or/and the control unit may comprise an electronic unit comprising as many channels as there are transducers, for example 3 channels, respectively connected to the transducers 107a;107b;107c. Each of these channels may comprise a converter analog-digital associated with a memory and communicating with a central unit electronics such as a microprocessor or similar, which itself can communicate for example with a memory and a signal processing circuit (such DSP), as well as with the computer 111b.

Figure 2a to 2d illustrate the method of the present disclosure in one or several embodiments.

Figure 2a illustrates the flowchart of the method according to the present disclosure.

In reference to figure 2a, the method for determining the velocity of a shear wave propagating in a medium may comprise:

- generating 201 at least one ultrasound beam 113a, 113b,113c into the medium illuminating at least three respective observation points S', M', P' in the medium;

- acquiring 203 a plurality of backscattered signals backscattered respectively from said observation points illuminated by said at least one ultrasound beam.

The ultrasound beam may be generated by using the detection system such this one presented in figure 1. For instance, in the case presented in the figures of the present disclosure, at least one ultrasound beam or a plurality of ultrasound beams may be generated by a plurality of ultrasound transducers located around a natural source 102 of shear waves, for instance on the surface of the medium as a chest of patient P and along a trajectory such the artery trajectory or myocardia trajectory of the patient P (i.e. where the wave vector direction of the shear wave is known), respectively at point S, M and P (or into the medium according to another example).

The shear wave may be generated by a natural source of shear waves comprising in the medium (or may be an external artificial source of shear waves). The natural source of shear waves may be comprised in an organ. For instance, the natural source of shear waves may be the movement of the closure valves of a heart of a patient.

Each ultrasound transducer 107a; 107b; 107c, located respectively at point S, M and P, may be configured to transmit ultrasound waves in the medium for forming (or generating) an respective ultrasound beam 113a;113b;113c, illuminating respective observations points S',M' and P' (or several observations points), and may be configured to acquire the respective backscattered signal from a respective observation point, the respective backscattered signals being generated in response to the interaction between an ultrasound beam 113a;113b;113c and the medium at this respective observation point.

. The backscattered signal associated to an observation point illuminated by an ultrasound beam may be retrieved (by signal processing for instance and carried out by the control system 111) from the totality of the backscattered signal emitted from the area (comprising the observation point) in the medium illuminated by the ultrasound beam.

Then, the method may comprise:

- determining a respective arrival time 207 for each observation 205 point based on the backscattered signals respectively acquired from the observation point.

Figure 2b illustrates the principle of detection of two arrival times.

In reference to Figure 2b, it is presented two variations of tissue velocity signals determined based on the backscattered signals respectively acquired from the observation points.

Each arrival time for a respective observation point may be determined by using a motion estimator on backscattered signals respectively acquired from this respective observation point.

In one or several embodiments, the motion estimator may be used to determine the velocity or the displacement of the tissue (or the variation of the velocity or the variation of the displacement of the tissue) from the backscattered signal backscattered from a respective observation point.

For instance, the tissue velocity (or the variation of the tissue velocity) may be determined by using the motion estimator on backscattered signals respectively acquired from a respective observation point S',M' by a respective ultrasound transducer 107a,107b, the respective ultrasound transducer may be the ultrasound transducer having generated the respective ultrasound beam illuminating the respective observation point (but not necessarily).

For instance, the variation 213a may be determined by computing the backscattered signal generated by the interaction of the ultrasound beam 113a with the medium 101 at the respective observation point S' (illuminated by the ultrasound beam 113a) and which have been acquired by the ultrasound transducer 107a. Likewise, the variation 213b may be determined by computing the backscattered signal generated by the interaction of the ultrasound beam 113b with the medium 101 at the respective observation point M' (illuminated by the ultrasound beam 113b) and which have been acquired by the ultrasound transducer 107b.

Each detected tissue velocity (or tissue displacement) variation 213a, 213b may reflect the passage of the shear wave at the respective observation point which is illuminated. Indeed, when a shear wave crosses a medium having a defined respective observation point, it modifies temporally the intrinsic properties of medium (e.g. physical properties), leading to a change of the backscattered signals monitored at this respective observation point, the backscattered signal resulting of the illumination, and therefore leading to a change of the computed tissue velocity at this respective observation point.

In one or several embodiments, each variation of the tissue velocity at a respective observation point may be computed from a plurality of images (e.g. 1D, 2D or 3D) constructed from the backscattered signals respectively acquired from this respective observation point. According to an example, the observation point may correspond to a pixel in a spatio-temporal image obtained based on a backscattered signals generated in response to respective ultrasound beams acquired at a given frame rate.

Thus, each curve of figure 2b may present a variation of an amplitude as a function in time of a tissue velocity signal computed for a respective observation point.

From these variations, it may be then possible to determine a respective arrival time at each observation point by detecting the maximum for each curve or by crossed correlation or any suitable known technique for instance. For instance, it may be possible to detect, for each variation, the maximum (e.g. maximal or minimal peak) or minimum. Thus, in reference to figure 2b, an arrival time $t_0$ may be detected in the variation 213a corresponding to its maximum, and an arrival time $t_1$ may be detected in the variation 213b corresponding to its maximum.

As the respective observation points are distinct and separated from each other, it may be possible to determine a delay, in time or phase, between the two detected arrival times.

In one or several embodiments, the arrival times may be detected directly from the plurality of backscattered signals by using artificial intelligence algorithm, and therefore, without the need to use the motion estimator (e.g. tissue velocity or tissue displacement).

Thus, in reference to figure 2b, it may be possible to determine a time delay $\Delta t$ between the two arrival times $t_0$ and $t_1$ detected following the respective maximum of each variation.

Such time delay may represent the time taken (e.g. time of flight) by the shear wave to propagate from the point S' to the point M' for a given velocity (or speed) in the medium.

Then, the method may comprise calculating 209 vector velocity components $(c_x, c_y, c_z)$ of the shear wave based on the plurality of respective arrival times.

In order to retrieve each component of the velocity of the shear wave (or each vector velocity component), such calculating may be carried, in one or several embodiments, as follows.

The figures 2c and 2d illustrate respectively the propagation of a shear wave in a medium according to a XY plane (plane perpendicular to the transmission direction for instance) and a XZ plane (comprising the transmission direction Z0 for instance).

In reference to figure 2c and 2d, the natural shear wave may be seen as a plane wave according to the following formula :

$$E = E_0 e^{i(wt - \vec{k}\vec{r})} \ (1)$$

The assumption of the wane plane may be true while the distance between the ultrasound beams of the plurality of ultrasound beam is less than the wavelength of the shear wave in the medium. For instance, the distance between the respective observation points or between the respective ultrasound transducers may be less than at 0.5 meters, preferably inferior at 0.3 meters (i.e. corresponding to a wavelength of the shear wave in the medium of 0.3 meter).

Such plane wave may be defined by a velocity c having three components $(c_x, c_y, c_z)$ (or three vector components) and a wave number $k(k_x, k_y, k_z)$.

The phase difference $\Delta\varphi$ between two plane waves defined by the equation (1) following an X axis may be written as follow :

$$\Delta\varphi = k\mathrm{x} \ (2)$$

From the generalization of this equation (2) in all directions (x, y, z), the phase difference between two variations of the tissue velocity signals at respective observation points S' and M' of the shear wave propagating in the medium and detected by the ultrasound transducers located at point S and point M may be written as follow:

$$\Delta\varphi_{S'M'} = \vec{k}\overrightarrow{S'M'} = k_x\overrightarrow{\Delta x_2 x_1} + k_y\overrightarrow{\Delta y_2 y_1} + k_z\overrightarrow{\Delta z_2 z_1} \ (3)$$

Likewise, the phase difference between two variations of the tissue velocity signals at respective observation points M' and P' of the shear wave propagating in the medium and detected by the ultrasound transducers located at point M and point P may be written as follows:

$$\Delta\varphi_{M'P'} = \vec{k}\overrightarrow{M'P'} = k_x\overrightarrow{\Delta x_3 x_2} + k_y\overrightarrow{\Delta y_3 y_2} + k_z\overrightarrow{\Delta z_3 z_2} \ (4)$$

At last, the phase difference between two variations of the tissue velocity signals at respective observation points S' and P' of the shear wave propagating in the medium and detected by the ultrasound transducers located at point S and point P may be written as follows:

$$\Delta\varphi_{S'P'} = \vec{k}\overrightarrow{S'P'} = k_x\overrightarrow{\Delta x_3 x_1} + k_y\overrightarrow{\Delta y_3 y_1} + k_z\overrightarrow{\Delta z_3 z_1} \ (5)$$

The equations (3), (4) and (5) may be written under matrix form as follows :

$$\begin{pmatrix} \Delta\varphi_{S'M'} \\ \Delta\varphi_{M'P'} \\ \Delta\varphi_{S'P'} \end{pmatrix} = \begin{bmatrix} \Delta x_2 x_1 & \Delta y_2 y_1 & \Delta z_2 z_1 \\ \Delta x_3 x_2 & \Delta y_3 y_2 & \Delta z_3 z_2 \\ \Delta x_3 x_1 & \Delta y_3 y_1 & \Delta z_3 z_1 \end{bmatrix} \begin{bmatrix} k_x \\ k_y \\ k_z \end{bmatrix} (6)$$

The phase difference $\Delta\varphi$ may be written in function of time delay $\Delta t$ according to $\Delta\varphi = \frac{T}{2\pi} * \Delta t$ with T is the period of the shear wave. By using equations $k = \frac{2\pi}{\lambda}$ and $\lambda = cT$, with c the velocity of the shear wave in the medium and $\lambda$ the wavelength of the shear wave, the matrix (6) may be written under the form :

$$\begin{pmatrix} \Delta t_{S'M'} \\ \Delta t_{M'P'} \\ \Delta t_{S'P'} \end{pmatrix} = \begin{bmatrix} \Delta x_2 x_1 & \Delta y_2 y_1 & \Delta z_2 z_1 \\ \Delta x_3 x_2 & \Delta y_3 y_2 & \Delta z_3 z_2 \\ \Delta x_3 x_1 & \Delta y_3 y_1 & \Delta z_3 z_1 \end{bmatrix} \begin{bmatrix} 1/c_x \\ 1/c_y \\ 1/c_z \end{bmatrix} (7)$$

At last, the equation (7) may be written under the following form and lead to :

$$\begin{bmatrix} 1/c_x \\ 1/c_y \\ 1/c_z \end{bmatrix} = \begin{bmatrix} \Delta x_2 x_1 & \Delta y_2 y_1 & \Delta z_2 z_1 \\ \Delta x_3 x_2 & \Delta y_3 y_2 & \Delta z_3 z_2 \\ \Delta x_3 x_1 & \Delta y_3 y_1 & \Delta z_3 z_1 \end{bmatrix}^{-1} \begin{pmatrix} \Delta t_{S'M'} \\ \Delta t_{M'P'} \\ \Delta t_{S'P'} \end{pmatrix} (8)$$

The equation (8) may therefore define the vector components ($c_x$, $c_y$, $c_z$) of the shear wave velocity as a function of delays along three perpendicular axes. This function depends on the plurality of delays and the positions of each observation point or lengths between each observation point.

Each delay (or time difference or time delay) $\Delta t_{S'M'}$, $\Delta t_{M'P'}$ and $\Delta t_{S'P'}$ may be known and may correspond to the time delays (e.g. time of flight) determined at the figure 2b and based on the plurality of arrival times detected in the variations of tissue velocity signals at respective observation points.

Likewise, according to figure 2c, the difference values along X and Y axis between two respective observation points may be known since the position of each ultrasound transducer is known. Indeed, as mentioned previously, the coordinates (x, y), in a plane perpendicular to the transmission direction for instance, for a respective observation point S', P', M' may correspond to the coordinates (x, y), in a plane perpendicular to the transmission direction for instance, of the position S, P, M of the ultrasound transducer 107a, 107b, 107c having acquired the plurality of backscattered signal at this respective position S', P', M' .

Thus, for instance, the values $\Delta x_2 x_1$ and $\Delta y_2 y_1$ between the observation points S' and M' may be determined based on the positions of the two ultrasound transducers respectively located at point S and point M which are known. Likewise, the values of $\Delta x_3 x_2$ and $\Delta y_3 y_2$ between the observation points M' and P' may be determined based on the positions of the two ultrasound transducers respectively located at point M and point P which are known. At last, the values of $\Delta x_3 x_1$ and $\Delta y_3 y_1$ between the observation points S' and P' may be determined based on the positions of the two ultrasound transducers respectively located at point S and point P (not represented on figure 2c) which are also known.

Likewise, the coordinate (z) of a respective observation point S', M', P' defined by a depth in the medium (for instance, along the transmission direction or along the central axis 120a;120b;120c of the ultrasound transducer S, M, P) is known, the values $\Delta z_2 z_1$, $\Delta z_3 z_2$ and $\Delta z_3 z_1$ may be also known. Indeed, the depth for each respective observation point is known in advance since defined by the detecting system for instance.

Besides, in one or several embodiments, the depth for an observation point may be swept along the central axis of the ultrasound transducer acquiring the backscattered signal backscattered from this observation point in order to get different variations of the backscattered signal for same coordinates x and y of the respective observation point (or of the position of the ultrasound transducer). Advantageously, the use of several depths for a same respective observation point may allow to increase the precision of the velocity's component along Z axis, and therefore, the precision of the velocity (or global velocity) of the shear wave.

In one or several embodiments, the depth for each respective observation point may be the same or may be different or may be the same for some respective observation point(s) and different for another. For instance, in reference to figure 2d, the respective observation point S' may have the same depth ($z_1 = z_2$) than the respective observation point M', and the respective observation point P' may have depth $z_3$ which is different that the depth of S' and/or M'.

Furthermore, it may be possible to increase the number of respective observation points by increasing the number of ultrasound transducers around/at proximity of the source of shear wave in order to increase the precision of the velocity's component along the X and Y axis and therefore, the precision of the velocity (or global velocity) of the shear wave.

Then the method may comprise an estimation 211 (or calculation) of the velocity (c) of the shear wave based on the calculated vector velocity components ($c_x$, $c_y$, $c_z$).

Such estimation may be carried out according to the following formula:

$$c = \frac{1}{\sqrt{\frac{1}{c_x^2} + \frac{1}{c_y^2} + \frac{1}{c_z^2}}} \quad (9)$$

From the calculation of the velocity (or global velocity) of the shear wave generated by the natural source of shear wave with the equation (9), it may be possible to determine the elastic properties of the medium, for instance biological tissues, around the natural source of shear waves. For instance, when the natural source of shear waves is a heart, it may be possible to determine the elastic properties of the biological tissues of the heart of the patient as well as the direction of cardiac muscle fibers at different depths for instance. The elastic properties may be, for instance, the Young modulus or/and the shear modulus of the medium (i.e. biological tissues).

Figure 3a and figure 3b present an example for measuring the velocity of a shear wave using a matrix ultrasound array probe classically used for imaging in 3D.

More precisely, figure 3a illustrates an experimental setup for measuring the velocity of a shear wave propagated in a medium and Figure 3b presents the results of the variations of tissue velocity signals determined based on the backscattered signals respectively acquired by the experimental setup from observation points.

In reference to figure 3a, the experimental setup 300 may comprise a matrix array probe 310 coupled with an ultrasound imaging system or a control system including for instance a control unit and a computer (not represented). The ultrasound array probe may comprise a plurality of transducers under a matrix form (e.g. 2D). The ultrasound probe may be located on the surface (or inside) of the medium 320, and some transducers of the matrix of transducers are used to generate respectively a plurality of ultrasound beams in the medium. Usually, such ultrasound probe is used in ultrasound imaging such 3D echography (or ultrasonography).

In this example, the medium 320 is a gelatin phantom, and at least one shear wave 330 is generated by a vibrating plate 340 at a frequency of 40 Hz placed inside the medium.

A few transducers of the ultrasound probe may be configured to transmit ultrasound waves in the medium for forming (or generating) ultrasound beams 350 used to illuminate nine observation points in the medium, and may be configured to acquire the backscattered signal from these observation points as described previously. The backscattered signal may be then processed by the experimental setup as described previously.

Figure 3b presents the results 360 of the variations of tissue velocity signals measured at the nine observation points with the experimental setup. As described for the figure 2b, each curve (of the nine curves) of figure 3b may present a variation of an amplitude as a function in time of a tissue velocity signal computed for a respective observation point of the nine observation points.

From these variations, it may be then possible to determine a respective arrival time at each observation point by detecting the maximum for each curve, and then to determine a time delay $\Delta t$ between two arrival times, and perform an inversion according to equation (8).

Based on the average of the determined time delays, the components of the shear wave may be calculated as described previously, and the velocity (or speed) of the shear wave may be determined by using an inversion method based on the calculated components of the shear wave.

In the case of this experimental setup, the velocity of the shear wave propagated in the medium is determined around 4.66 m/s.

Therefore, advantageously, the present method may use existing (or conventional) ultrasound imaging comprising conventional ultrasound probe or using imaging system using conventional ultrasound probe (having ultrasound transducers) for determining the velocity of shear wave propagating in a medium (e.g. biological tissues of the heart).

**Claims**

1. A method for determining a velocity of a shear wave (105) propagating in a medium (101), said shear wave having a wavelength, the method comprising:

   - generating (201) at least one ultrasound beam (113a, 113b,113c) into the medium illuminating at least three respective observation points (S', M', P') in the medium;

- acquiring (203) a plurality of backscattered signals backscattered respectively from said observation points illuminated by said at least one ultrasound beam;
- determining (207) a respective arrival time for each observation (205) point based on the backscattered signals respectively acquired from the observation point;
- calculating (209) vector velocity components ($c_x$, $c_y$, $c_z$) of the shear wave based on the plurality of respective arrival times;
- estimating (211) the velocity (c) of the shear wave based on the calculated vector velocity components ($c_x$, $c_y$, $c_z$);

wherein the respective observation points are distinct and are not aligned.

2. Method according to claim 1, wherein at least three ultrasound beams (113a, 113b, 113c) are generated into the medium illuminating respectively the at least three respective observation points (S', M', P') in the medium.

3. Method according to claim 1 or claim 2, wherein the plurality of backscattered signals is respectively acquired by a plurality of ultrasound transducers, each ultrasound transducer acquiring backscattered signals backscattered respectively from a respective observation point, preferably,
the at least three ultrasound beams (113a, 113b, 113c) are generated respectively by the plurality of ultrasound transducers.

4. Method according to claim 3, wherein the at least three ultrasound beams (113a, 113b, 113c) are generated respectively by at least three ultrasound transducers of the plurality of ultrasound transducers.

5. Method according to any one of the preceding claims, wherein the respective observation points are separated from each other by a separation distance less than the wavelength of the shear wave in the medium, preferably,
the wavelength of the shear wave is less than 0.7 meters, preferably less than 0.5 meters.

6. Method according to any one of the preceding claims, wherein when the wave vector direction of the shear wave is known, the at least three observation points may be not aligned in the plane that contains the wave vector of the shear wave, or when the wave vector direction of the shear wave is unknown, the at least three observation points may be at least four observation points which are not aligned and not all comprised in a same plane.

7. Method according to any one of the preceding claims 1-6, wherein each arrival time of the plurality of respective arrival times for a respective observation point is determined using a motion estimator on backscattered signals respectively acquired from this respective observation point,
preferably, each arrival time of the plurality of respective arrival times for a respective observation point is determined based on a respective variation of a tissue velocity determined by the motion estimator at this respective observation point.

8. Method according to any one of the preceding claims, wherein calculating the vector velocity components of the shear wave further comprises a determination of a plurality of at least three delays, each delay ($\Delta t$) being determined between two respective arrival times of the plurality of arrival times, and each vector velocity component being function of a respective delay ($\Delta t$) of the plurality of at least three delays.

9. Method according to any one of the preceding claims 7-8, wherein each arrival time of a respective observation point determined based on the respective variation of the tissue velocity at this respective observation point is determined by detecting a maximum and/or a minimum of the respective variation or by using crossed correlation or artificial intelligence algorithm.

10. Method according to any one of the preceding claims, wherein estimating of the velocity of the shear wave based on the calculated vector velocity components is performed by using inversion algorithm on the calculated vector velocity components.

11. Method according to any one of the preceding claims, wherein the shear wave is a natural shear wave generated by a natural source of shear waves comprised in the medium or is an artificial shear wave generated by an artificial source of shear waves outside the medium, preferably, the natural source of shear waves is a movement of closure valves of a heart or vibration caused by voice or pulse wave propagating in the arterial wall.

12. Method according to any one of the preceding claims 3-4, wherein the plurality of ultrasound transducers is located on the surface of the medium and/or into the medium.

13. A detection system (100) for determining a velocity of a shear wave (105) propagating in a medium (101) using a method according to claims 1-12, said detection system comprises:

   - a plurality of ultrasound transducers arranged in a 2D array (107a, 107b, 107c) for generating at least one ultrasound beam (113a, 113b,113c) into the medium and illuminating at least three respective observation points (S', M', P') in the medium, and being configured to acquire a respective backscattered signal generated in response to an interaction between the at least one ultrasound beam and the medium,
   - a control unit configured for having the ultrasound transducers of the plurality of ultrasound transducers for generating the at least one ultrasound beam, for acquiring a plurality of backscattered signals backscattered respectively from said at least three respective observation points illuminated by said at least one ultrasound beam, for determining a respective arrival time for each observation point based on the backscattered signals respectively acquired from the observation point, for calculating (209) vector velocity components ($c_x$, $c_y$, $c_z$) of the shear wave based on the plurality of respective arrival times and for estimating (211) the velocity (c) of the shear wave based on the calculated vector velocity components ($c_x$, $c_y$, $c_z$);

   wherein the respective observation points are distinct and are not aligned.

14. Detection system according to claim 13, wherein at least three ultrasound beams (113a, 113b,113c) are generated into the medium illuminating respectively the at least three respective observation points (S', M', P') in the medium.

15. Detection system according to claim 13-14, wherein each ultrasound transducer of the plurality of ultrasound transducers acquires backscattered signals backscattered respectively from a respective observation point.

16. Detection system according to any one of the preceding claims 13-15, wherein each ultrasound transducer is formed by a matrix of transducer elements adapted to be controlled independently, and the control system may be configured to control the ultrasound transducer elements so that to emulate an ultrasound transducer generating an ultrasound beam in the medium.

**Patentansprüche**

1. Verfahren zum Bestimmen der Geschwindigkeit einer Scherwelle (105), die sich in einem Medium (101) ausbreitet, wobei die Scherwelle eine Wellenlänge aufweist, wobei das Verfahren Folgendes umfasst:

   - Erzeugen (201) mindestens eines Ultraschallstrahls (113a, 113b, 113c) in das Medium, der mindestens drei jeweilige Beobachtungspunkte (S', M', P') in dem Medium beleuchtet;
   - Erfassen (203) einer Vielzahl von rückgestreuten Signalen, die jeweils von den durch den mindestens einen Ultraschallstrahl beleuchteten Beobachtungspunkten zurückgestreut werden;
   - Bestimmen (207) einer jeweiligen Ankunftszeit für jeden Beobachtungspunkt (205) basierend auf den jeweils von dem Beobachtungspunkt erfassten rückgestreuten Signalen;
   - Berechnen (209) der Vektorgeschwindigkeitskomponenten ($c_x$, $c_y$, $c_z$) der Scherwelle basierend auf der Vielzahl von jeweiligen Ankunftszeiten;
   - Schätzen (211) der Geschwindigkeit (c) der Scherwelle basierend auf den berechneten Vektorgeschwindigkeitskomponenten ($c_x$, $c_y$, $c_z$);

   wobei die jeweiligen Beobachtungspunkte unterschiedlich sind und nicht ausgerichtet sind.

2. Verfahren nach Anspruch 1, wobei mindestens drei Ultraschallstrahlen (113a, 113b, 113c) in das Medium erzeugt werden, die jeweils die mindestens drei jeweiligen Beobachtungspunkte (S', M', P') in dem Medium beleuchten.

3. Verfahren nach Anspruch 1 oder 2, wobei die Vielzahl von rückgestreuten Signalen jeweils von einer Vielzahl von Ultraschallwandlern erfasst wird, wobei jeder Ultraschallwandler jeweils rückgestreute Signale von einem jeweiligen Beobachtungspunkt erfasst,
wobei vorzugsweise mindestens drei Ultraschallstrahlen (113a, 113b, 113c) jeweils von der Vielzahl von Ultraschallwandlern erzeugt werden.

4. Verfahren nach Anspruch 3, wobei die mindestens drei Ultraschallstrahlen (113a, 113b, 113c) jeweils von mindestens drei Ultraschallwandlern aus der Vielzahl von Ultraschallwandlern erzeugt werden.

5. Verfahren nach einem der vorhergehenden Ansprüche, wobei die jeweiligen Beobachtungspunkte durch einen Trennabstand voneinander getrennt sind, der kleiner ist als die Wellenlänge der Scherwelle in dem Medium, wobei vorzugsweise die Wellenlänge der Scherwelle weniger als 0,7 Meter, vorzugsweise weniger als 0,5 Meter, beträgt.

6. Verfahren nach einem der vorhergehenden Ansprüche, wobei, wenn die Wellenvektorrichtung der Scherwelle bekannt ist, die mindestens drei Beobachtungspunkte nicht in der Ebene ausgerichtet sein können, die den Wellenvektor der Scherwelle enthält, oder wenn die Wellenvektorrichtung der Scherwelle unbekannt ist, die mindestens drei Beobachtungspunkte mindestens vier Beobachtungspunkte sein können, die nicht ausgerichtet sind und nicht alle in einer Ebene enthalten sind.

7. Verfahren nach einem der vorhergehenden Ansprüche 1 bis 6, wobei jede Ankunftszeit der Vielzahl von jeweiligen Ankunftszeiten für einen jeweiligen Beobachtungspunkt unter Verwendung eines Bewegungsschätzers auf rückgestreuten Signalen, die jeweils von diesem jeweiligen Beobachtungspunkt erfasst werden, bestimmt wird, wobei vorzugsweise jede Ankunftszeit der Vielzahl von jeweiligen Ankunftszeiten für einen jeweiligen Beobachtungspunkt basierend auf einer jeweiligen Variation einer durch den Bewegungsschätzer an diesem jeweiligen Beobachtungspunkt bestimmten Gewebegeschwindigkeit bestimmt wird.

8. Verfahren nach einem der vorhergehenden Ansprüche, wobei das Berechnen der Vektorgeschwindigkeitskomponenten der Scherwelle ferner das Bestimmen einer Vielzahl von mindestens drei Verzögerungen umfasst, wobei jede Verzögerung ($\Delta t$) zwischen zwei jeweiligen Ankunftszeiten der Vielzahl von Ankunftszeiten bestimmt wird und jede Vektorgeschwindigkeitskomponente eine Funktion einer jeweiligen Verzögerung ($\Delta t$) aus der Vielzahl von mindestens drei Verzögerungen ist.

9. Verfahren nach einem der vorhergehenden Ansprüche 7 bis 8, wobei jede Ankunftszeit eines jeweiligen Beobachtungspunkts, die basierend auf der jeweiligen Variation der Gewebegeschwindigkeit an diesem Beobachtungspunkt bestimmt wird, durch das Detektieren eines Maximums und/oder eines Minimums der jeweiligen Variation oder durch die Verwendung von Kreuzkorrelation oder eines künstlichen Intelligenzalgorithmus bestimmt wird.

10. Verfahren nach einem der vorhergehenden Ansprüche, wobei das Schätzen der Geschwindigkeit der Scherwelle basierend auf den berechneten Vektorgeschwindigkeitskomponenten unter Verwendung eines Inversionsalgorithmus auf den berechneten Vektorgeschwindigkeitskomponenten durchgeführt wird.

11. Verfahren nach einem der vorhergehenden Ansprüche, wobei die Scherwelle entweder eine natürliche Scherwelle, die von einer natürlichen Quelle von Scherwellen in dem Medium erzeugt wird, oder eine künstliche Scherwelle ist, die von einer künstlichen Quelle von Scherwellen außerhalb des Mediums erzeugt wird, wobei vorzugsweise die natürliche Quelle von Scherwellen eine Bewegung der Verschlussklappen eines Herzens oder eine Vibration ist, die durch Stimm- oder Pulswellen verursacht wird, die sich in der Arterienwand ausbreiten.

12. Verfahren nach einem der vorhergehenden Ansprüche 3 bis 4, wobei sich die Vielzahl von Ultraschallwandlern auf der Oberfläche des Mediums und/oder in dem Medium befindet.

13. Detektionssystem (100) zum Bestimmen der Geschwindigkeit einer Scherwelle (105), die sich in einem Medium (101) ausbreitet, unter Verwendung eines Verfahrens nach den Ansprüchen 1 bis 12, wobei das Detektionssystem Folgendes umfasst:

- eine Vielzahl von Ultraschallwandlern, die in einem 2D-Array (107a, 107b, 107c) angeordnet sind, um mindestens einen Ultraschallstrahl (113a, 113b, 113c) in das Medium zu erzeugen und mindestens drei jeweilige Beobachtungspunkte (S', M', P') in dem Medium zu beleuchten, und die so konfiguriert sind, dass sie ein jeweiliges rückgestreutes Signal erfassen, das als Reaktion auf eine Interaktion zwischen dem mindestens einen Ultraschallstrahl und dem Medium erzeugt wird,
- eine Steuereinheit, die so konfiguriert ist, dass sie die Ultraschallwandler der Vielzahl von Ultraschallwandlern verwendet, um den mindestens einen Ultraschallstrahls zu erzeugen, um eine Vielzahl von rückgestreuten Signalen zu erfassen, die jeweils von den mindestens drei jeweiligen Beobachtungspunkten, die von dem mindestens einen Ultraschallstrahl beleuchtet werden, zurückgestreut werden, um eine jeweilige Ankunftszeit für jeden Beobachtungspunkt basierend auf den jeweils von dem Beobachtungspunkt erfassten rückgestreuten Signalen zu bestimmen, um die Vektorgeschwindigkeitskomponenten ($c_x$, $c_y$, $c_z$) der Scherwelle basierend auf der Vielzahl von jeweiligen Ankunftszeiten zu berechnen (209) und um die Geschwindigkeit (c) der Scherwelle basierend auf den berechneten Vektorgeschwindigkeitskomponenten ($c_x$, $c_y$, $c_z$) zu schätzen (211);

wobei die jeweiligen Beobachtungspunkte unterschiedlich sind und nicht ausgerichtet sind.

14. Detektionssystem nach Anspruch 13, wobei mindestens drei Ultraschallstrahlen (113a, 113b, 113c) in das Medium erzeugt werden, die jeweils die mindestens drei jeweiligen Beobachtungspunkte (S', M', P') in dem Medium beleuchten.

15. Detektionssystem nach Anspruch 13 bis 14, wobei jeder Ultraschallwandler der Vielzahl von Ultraschallwandlern rückgestreute Signale erfasst, die jeweils von einem jeweiligen Beobachtungspunkt zurückgestreut werden.

16. Detektionssystem nach einem der vorhergehenden Ansprüche 13 bis 15, wobei jeder Ultraschallwandler aus einer Matrix von Schallwandlerelementen besteht, die so ausgelegt sind, dass sie unabhängig voneinander gesteuert werden können, und das Steuersystem so konfiguriert sein kann, dass es die Ultraschallwandlerelemente steuert, um einen Ultraschallwandler zu emulieren, der einen Ultraschallstrahl in dem Medium erzeugt.

**Revendications**

1. Procédé de détermination d'une vitesse d'une onde de cisaillement (105) se propageant dans un milieu (101), ladite onde de cisaillement ayant une longueur d'onde, le procédé comprenant :

   - la génération (201) d'au moins un faisceau d'ultrasons (113a, 113b, 113c) dans le milieu, éclairant au moins trois points d'observation (S', M', P') respectifs dans le milieu ;
   - l'acquisition (203) d'une pluralité de signaux rétrodiffusés provenant respectivement desdits points d'observation éclairés par ledit au moins un faisceau d'ultrasons ;
   - la détermination (207) d'un temps d'arrivée respectif pour chaque point d'observation (205) sur la base des signaux rétrodiffusés acquis respectivement à partir du point d'observation ;
   - le calcul (209) des composantes de vitesse vectorielle ($c_x$, $c_y$, $c_z$) de l'onde de cisaillement sur la base de la pluralité des temps d'arrivée respectifs ;
   - l'estimation (211) de la vitesse (c) de l'onde de cisaillement sur la base des composantes de vitesse vectorielle ($c_x$, $c_y$, $c_z$) calculées ;

   dans lequel les points d'observation respectifs sont distincts et non alignés.

2. Procédé selon la revendication 1, dans lequel au moins trois faisceaux d'ultrasons (113a, 113b, 113c) sont générés dans le milieu, éclairant respectivement les au moins trois points d'observation (S', M', P') respectifs dans le milieu.

3. Procédé selon la revendication 1 ou la revendication 2, dans lequel la pluralité de signaux rétrodiffusés est respectivement acquise par une pluralité de transducteurs ultrasonores, chaque transducteur ultrasonores acquérant des signaux rétrodiffusés provenant respectivement d'un point d'observation respectif, de préférence, les au moins trois faisceaux d'ultrasons (113a, 113b, 113c) sont générés respectivement par la pluralité de transducteurs ultrasonores.

4. Procédé selon la revendication 3, dans lequel les au moins trois faisceaux d'ultrasons (113a, 113b, 113c) sont générés respectivement par au moins trois transducteurs ultrasonores de la pluralité de transducteurs ultrasonores.

5. Procédé selon l'une quelconque des revendications précédentes, dans lequel les points d'observation respectifs sont séparés les uns des autres par une distance de séparation inférieure à la longueur d'onde de l'onde de cisaillement dans le milieu, de préférence, la longueur d'onde de l'onde de cisaillement est inférieure à 0,7 mètre, de préférence inférieure à 0,5 mètre.

6. Procédé selon l'une quelconque des revendications précédentes, dans lequel lorsque la direction du vecteur d'onde de l'onde de cisaillement est connue, les au moins trois points d'observation peuvent ne pas être alignés dans le plan qui contient le vecteur d'onde de l'onde de cisaillement, ou lorsque la direction du vecteur d'onde de l'onde de cisaillement est inconnue, les au moins trois points d'observation peuvent être au moins quatre points d'observation qui ne sont pas alignés et pas tous compris dans un même plan.

7. Procédé selon l'une quelconque des revendications 1 à 6, dans lequel chaque temps d'arrivée de la pluralité de temps d'arrivée respectifs pour un point d'observation respectif est déterminé à l'aide d'un estimateur de mouvement sur des signaux rétrodiffusés acquis respectivement à partir de ce point d'observation, de préférence, chaque temps d'arrivée parmi la pluralité de temps d'arrivée respectifs pour un point d'observation respectif est déterminé sur la base d'une variation respective d'une vitesse de tissu déterminée par l'estimateur de mouvement à ce point d'observation respectif.

8. Procédé selon l'une quelconque des revendications précédentes, dans lequel le calcul des composantes de vitesse vectorielle de l'onde de cisaillement comprend en outre la détermination d'une pluralité d'au moins trois retards, chaque retard ($\Delta t$) étant déterminé entre deux temps d'arrivée respectifs de la pluralité de temps d'arrivée, et chaque composante de vitesse vectorielle étant fonction d'un retard ($\Delta t$) respectif parmi la pluralité d'au moins trois retards.

9. Procédé selon l'une quelconque des revendications 7 à 8 précédentes, dans lequel chaque temps d'arrivée d'un point d'observation respectif déterminé sur la base de la variation respective de la vitesse de tissu à ce point d'observation respectif est déterminé en détectant un maximum et/ou un minimum de la variation respective ou en utilisant une corrélation croisée ou un algorithme d'intelligence artificielle.

10. Procédé selon l'une quelconque des revendications précédentes, dans lequel l'estimation de la vitesse de l'onde de cisaillement sur la base des composantes de vitesse vectorielle calculées est réalisée en utilisant un algorithme d'inversion sur les composantes de vitesse vectorielle calculées.

11. Procédé selon l'une quelconque des revendications précédentes, dans lequel l'onde de cisaillement est une onde de cisaillement naturelle générée par une source naturelle d'ondes de cisaillement présente dans le milieu ou est une onde de cisaillement artificielle générée par une source artificielle d'ondes de cisaillement située en dehors du milieu, de préférence, la source naturelle d'ondes de cisaillement est un mouvement des valves de fermeture d'un cœur ou une vibration provoquée par la propagation d'une voix ou d'une onde de pouls dans la paroi artérielle.

12. Procédé selon l'une quelconque des revendications 3 et 4 précédentes, dans lequel la pluralité de transducteurs ultrasonores est située à la surface du milieu et/ou dans le milieu.

13. Système de détection (100) pour déterminer une vitesse d'une onde de cisaillement (105) se propageant dans un milieu (101) en utilisant un procédé selon les revendications 1 à 12, ledit système de détection comprenant :

   - une pluralité de transducteurs ultrasonores agencés selon un réseau 2D (107a, 107b, 107c) pour générer au moins un faisceau d'ultrasons (113a, 113b, 113c) dans le milieu et éclairer au moins trois points d'observation (S', M', P') respectifs dans le milieu, et étant configurés pour acquérir un signal rétrodiffusé respectif généré en réponse à une interaction entre l'au moins un faisceau d'ultrasons et le milieu,
   - une unité de commande configurée pour utiliser les transducteurs ultrasonores de la pluralité de transducteurs ultrasonores afin de générer l'au moins un faisceau d'ultrasons, pour acquérir une pluralité de signaux rétrodiffusés provenant respectivement desdits au moins trois points d'observation respectifs éclairés par ledit au moins un faisceau d'ultrasons, pour déterminer un temps d'arrivée respectif pour chaque point d'observation sur la base des signaux rétrodiffusés respectivement acquis à partir du point d'observation, pour calculer (209) des composantes de vitesse vectorielle ($c_x$, $c_y$, $c_z$) de l'onde de cisaillement sur la base de la pluralité de temps d'arrivée respectifs et pour estimer (211) la vitesse (c) de l'onde de cisaillement sur la base des composantes de vitesse vectorielle ($c_x$, $c_y$, $c_z$) calculées ;

   dans lequel les points d'observation respectifs sont distincts et non alignés.

14. Système selon la revendication 13, dans lequel au moins trois faisceaux d'ultrasons (113a, 113b, 113c) sont générés dans le milieu, éclairant respectivement les au moins trois points d'observation (S', M', P') respectifs dans le milieu.

15. Système de détection selon les revendications 13 et 14, dans lequel chaque transducteur ultrasonore de la pluralité de transducteurs ultrasonores acquiert des signaux rétrodiffusés provenant respectivement d'un point d'observation respectif.

16. Système de détection selon l'une quelconque des revendications 13 à 15 précédentes, dans lequel chaque transducteur ultrasonore est formé par une matrice d'éléments transducteurs adaptés pour être commandés indépendamment, et le système de commande peut être configuré pour commander les éléments transducteurs ultrasonores de manière à émuler un transducteur ultrasonore générant un faisceau d'ultrasons dans le milieu.

FIG. 1

```
┌─────────────────────────┐
│        gen_be           │──── 201
└─────────────────────────┘
             │
             ▼
┌─────────────────────────┐
│     Acq_bck_signal      │──── 203
└─────────────────────────┘
             │
             ▼
┌─────────────────────────┐
│      For_each_obs       │──── 205
└─────────────────────────┘
   ▲         │
   │         ▼
   │  ┌─────────────────────────┐
   └──│     Det_arriv_tim       │──── 207
      └─────────────────────────┘
                 │
                 ▼
      ┌─────────────────────────┐
      │    Calc_spd_compo       │──── 209
      └─────────────────────────┘
                 │
                 ▼
      ┌─────────────────────────┐
      │       Estim_spd         │──── 211
      └─────────────────────────┘
```

# FIG. 2a

FIG. 2b

FIG. 2c

FIG. 2d

**FIG. 3a**

FIG. 3b

# EP 4 510 938 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Three-dimensional shear wave elastography on conventional ultrasound scanners with external vibration. **CHENGWU HUANG et al.** Physics in Medicine and Biology. Institute of Physics Publishing, vol. 65, 215009